# NEUE EUROPÄISCHE PATENTSCHRIFT

(11) **EP 0 859 751 B2**
(45) Veröffentlichungstag und Bekanntmachung des Hinweises auf die Entscheidung über den Einspruch: **18.07.2007**
(45) Hinweis auf die Patenterteilung: 19.01.2000
(21) Anmeldenummer: 96927711.0
(22) Anmeldetag: 09.08.1996
(51) Int. Cl.: C07C 19/08, A61K 31/02, C10M 105/52

(54) **SEMIFLUORIERTE ALKANE UND IHRE VERWENDUNG**
SEMI-FLUORINATED ALKANES AND THEIR USE
ALCANES SEMI-FLUORES ET LEUR UTILISATION

(30) Priorität: 29.09.1995 DE 19536504
(43) Veröffentlichungstag der Anmeldung: 26.08.1998
(60) Teilanmeldung: 99105854.6
(73) Patentinhaber: Meinert, Hasso, 89231 Neu-Ulm (DE)
(72) Erfinder: Meinert, Hasso, 89231 Neu-Ulm (DE)
(74) Vertreter: Nöth, Heinz
(86) Internationale Anmeldenummer: PCT/EP1996/003542
(87) Internationale Veröffentlichungsnummer: WO 1997/012852

(56) Entgegenhaltungen:
- EP-A- 0 444 752
- EP-A- 0 563 446
- WO-A.-93/01798
- DE-A- 3 816 467
- DE-A- 3 925 525
- US-A- 2 997 505
- CHEMICAL ABSTRACTS, vol. 101, no. 2, 9.Juli 1984 Columbus, Ohio, US; abstract no. 007909, TWIEG R ET AL: "Synthesis and characterization of perfluoroalkylalkanes (PFAA) as models for semiflexible polymers" XP002022708 & POLYM. PREPR. (AM. CHEM. SOC., DIV. POLYM. CHEM.) (ACPPAY,00323934);84; VOL.25 (1); PP.154-5, IBM RES. LAB.;SAN JOSE; 95193; CA; USA (US),
- Klin. Oczna 93, S. 105-107, 1993
- H. Meinert, Konferenzband JERMOV 97, Montpellier, 15.-19. Okt., Frankreich
- Medizinprodukte, Normensammlung C (EN 30993-1, 1994), S. 6-8
- Biomat Art. Cells & Immob. Biotech, 21(5), 583-595, 1993
- Der Augenspiegel, 12, 2000, 28-32
- Int, Ophthalmology, 23, 1999, 17-24
- Seminars in Ophthalmology, 15, 2000, 25-35
- D. Zeana et al, 2000 ASCRS and ESCRS
- B. Kirchhof et al, Europ. Multicentre Randomized Contr. Clin. Trail Version 1.0.0., 19.5.2000
- B. Kirchhof et al, Multizentrische Studien, RWTH Aachen, 2.12.1999
- Amer. J. Ophthalmology, 2001, in press
- Ophthalmologe 9, 2000, 652-661
- Ophthalmo-Chirurgie, 11, 1999, 153-158
- Graefe's Arch. Clin. Exp. Ophthalmol. 237, 1999, 201-206
- Ocular Surgery News, 20.03.2000
- Ocular Surgery News, 15.02.1999
- Eur. J. Ophthalmology, 10, 2000, 189-197

## Beschreibung

Die Erfindung betrifft die Verwendung semifluorierter Alkane der allgemeinen Formeln

R_{F}R_{H}

wobei R_{F} eine lineare oder verzweigte Perfluoralkyl-Gruppe und R_{H} eine lineare oder verzweigte, gesättigte (Kohlenwasserstoff)-Alkyl-Gruppe ist, für die Augenheilkunde.

Die unverzweigten semifluorierten Alkane besitzen die Formeln

F(CF₂)ₙ(CH₂)ₘH

mit n = 3 - 20
m = 3 - 20

Die verzweigten semifluorierten Alkane können innerhalb der Perfluoralkyl-Gruppen auch - FCX- -Einheiten
mit
X = C₂F₅, C₃F₇ oder C₄F₉
sowie innerhalb der Alkyl-Gruppen auch - HCY- -Einheiten
mit
Y = C₂H₅, C₃H₇ oder C₄H₉
enhalten.

Innerhalb einer Perfluoralkyl-Kette kann auch eine -CX₂- -Gruppe, sowie innerhalb einer Alkyl-Kette auch eine -CY₂- -Gruppe enthalten sein.

Endständig im Molekül kann anstelle der Perfluoralkyl-Gruppe F₃C- auch eine
FCX₂- oder F₂CX--Gruppe mit
X = C₂F₅, C₃F₇ oder C₄F₉
gebunden sein und ebenso kann endständig im Molekül anstelle der Alkyl-Gruppe H₃C- auch eine HCY₂- oder H₂CY--Gruppe
mit
Y = C₂H₅, C₃H₇ oder C₄H₉
gebunden sein.

Stets aber bleibt im Falle aller genannten Isomeren, das heißt linearen oder verzweigten semifluorierten Alkane die Gesamtzahl der Kohlenstoffatome im Perfluoralkyl-Teil wie vorgegeben in den Grenzen von n = 3 - 20. Auch bleibt im Alkyl-Teil die Zahl der Kohlenstoffatome in den vorgegebenen Grenzen von m = 3 - 20.

Diese semifluorierten Alkane, kurz auch als Diblock-Verbindungen R_{F}R_{H} bezeichnet, können sowohl in den Perfluoralkyl-Gruppen als auch in den Alkylgruppen linear oder verzweigt sein, wobei im Falle der Isomeren die Gesamtzahl der Kohlenstoffatome im Perfluoralkylteil in den vorgegebenen Grenzen von n = 3 - 20 und im Alkylteil die Gesamtzahl der Kohlenstoffatome in den vorgegebenen Grenzen von m = 3- 20 bleibt.

Diese Verbindungs-Typen sind analog den Perfluorcarbonen (Verbindungen, die nur aus Kohlenstoff-Fluor-Bindungen bestehen) chemisch, physikalisch und physiologisch inert und somit untoxisch.
Gegenüber den Perfluorcarbonen sind die semifluorierten Diblock-Alkane völlig anders aufgebaut. Sie bestehen aus geschlossenen Kohlenwasserstoff-Alkan-Gruppen, -(CH₂)ₙ- bzw. -(CHR_{H})ₙ-, bzw. -(CH₂)ₙH, die direkt an Perfluoralkyl-Gruppen, -(CF₂)ₘ- bzw. -(CFR_{F})- bzw. -(CF₂)F gebunden sind. Dabei kann der Perfluoralkyl-Teil auch mit einer (CR_{F}R_{F}F)-Gruppierung beginnen und mit einer (CR_{H}R_{H}H)-Gruppierung enden. Innerhalb einer Perfluoralkyl-Kette kann auch eine -R_{F}CR_{F}- -Gruppe, sowie innerhalb einer Alkyl-Kette kann auch eine
-R_{H}CR_{H}-.-Gruppe gebunden sein.

Bei diesen Verbindungs-Typen kann eine intramolekulare HF-Abspaltung unter Ausbildung fluorolefinischer Doppelbindungen nicht stattfinden. Im Gegenteil, die geschlossene Kohlenwasserstoff-Alkan-Gruppierung wirkt noch bindungsverstärkend auf die ohnehin sehr festen C-F-Bindungen im Perfluoralkyl-Teil der jeweiligen Verbindung.
Die semifluorierten Diblock-Alkane sind farblose Flüssigkeiten oder Festkörper. Sie werden nicht von starken Säuren oder Laugen oder Oxidationsmitteln oder Nukleophilen angegriffen, ebensowenig findet ein metabolischer oder katabolischer Angriff statt.
Auch physikalisch verhalten sich die semifluorierten Alkane wie modifizierte Perfluorcarbone, wobei mit zunehmender Molekülmasse die Siedepunkte und Schmelzpunkte ansteigen (s. Tab. 1, 2). Semifluorierte Alkane besitzen ähnlich den reinen Perfluorcarbonen eine hohe Löslichkeit für Gase, u.a. für O₂ und CO₂ (ca. 40-50 bzw. 130-150 vol.%).
Analog den Perfluorcarbonen und den Kohlenwasserstoffen sind die semifluorierten Alkane in Wasser kaum bis nicht löslich.
Jedoch lassen sie sich mittels wirksamer nichtionischer Tenside (Fluortenside, Verbindungen mit fluorophilem Kopf und hydrophilem Schwanz, Ethylenoxid-Propylenoxid-Blockpolymere, Pluronics® oder Phospholipide, wie Eilezithin oder Sojalezithin, u.a.) oder ionischer Tenside unter Zuhilfenahme von physikalischen Emulgierungsverfahren (Ultraschall- bzw. Gaulin-Homogenisatoren) in teilchenstabile Emulsionen mit Partikelgrößen von ca. 100-300 nm analog [1] überführen.
Zum Unterschied zu den Perfluorcarbonen sind die semifluorierten Alkane, abhängig vom jeweiligen R_{F}- bzw. R_{H}-Anteil, sowohl in Perfluorcarbonen und Derivaten von Perfluorcarbonen (höhermolekulare perfluorierte Ether, Hostinert^{®}, Fomblin^{®} u.a.) [1-4] als auch in Kohlenwasserstoffen und deren Derivaten und dabei Verbindungen mit höheren Alkylgruppen-Anteilen (z.B. flüssige Paraffine, Silikonöle, Fettsäure-Ester u.a.) löslich. Dabei steigt mit steigendem Perfluoralkyl-Anteil die Löslichkeit in den Fluorcarbon-Systemen, während mit steigendem Alkyl-Anteil die Löslichkeit in den Kohlenwasserstoff-Systemen zunimmt, und umgekehrt.
In Lösungen von semifluorierten Alkanen, insbesondere von semifluorierten lineraren Alkanen, in Kohlenwasserstoffen können lamellare Doppelschichten vorliegen, wobei unter bestimmten Voraussetzungen, z.B. in Abhängigkeit vom Konzentrationsverhältnis oder beim Abkühlen, die zuvor homogenen, optisch klaren Lösungen in opake Gele übergehen. Beim nachträglichen Erwärmen werden wieder die homogenen Lösungen erhalten. Die Gelbildung beruht darauf, daß die Lösungsmittel-Kohlenwasserstoffe von den Diblock-Doppelschichten der R_{F}R_{H}s aufgenommen werden [3,4].
Im Gegensatz zu den Perfluorcarbonen (Dichten 1,8 - 2,0 g/cm³) liegen die Dichten der semifluorierten Alkane mit Werten zwischen 1,1 bis 1,7 g/cm³ wesentlich niedriger, dadurch bedingt, daß diese Moleküle einen hohen Anteil an Kohlenwasserstoff-Gruppierungen, enthalten.
Andererseits besitzen die vorgeschlagenen Verbindungen die vorzüglichen Eigenschaften der Perfluorcarbone hinsichtlich ihrer Grenzflächenspannung gegen Wasser (R_{F}R_{H} 50-58 mN/m bei 20 °C) und der außerordenlich niedrigen Oberflächenspannung (R_{F}R_{H} 15-22 mN/m bei 20 °C), dadurch bedingt, daß am Molekülende Perfluoralkylgruppen gebunden sind.
Wenn in Lösungen der zuvorgenannten Kohlenwasserstoffe und deren Derivaten mit semi-fluorierten Diblock- oder Triblock-Alkanen aufgrund von relativ hohen Dichteunterschieden (dann gegeben, wenn semifluorierte Alkane mit hohem Perfluoralkylanteil verwendet werden) bei längerem Stehen eine Entmischung eintritt, läßt sich durch einfaches Schütteln oder leichtes Erzwärmen die homogene, optisch klare Lösung wieder herstellen.
Die semifluorierten Diblock-Alkane werden durch Umsetzung von Perfluoralkyliodiden mit Alkenen oder α,ω-Dienen, nach HI-Eliminierung und abschließender Hydrierung mittels H₂/Raney-Nickel im Hochdruckverfahren [5] oder durch Hydrierung mittels Platin-Kontakt [6] oder mittels Tributylzinnhydrid [4] gewonnen (s. Anhang: Beispiele für Syntheses semifluorierter Diblock- und Triblockalkane).
Die dabei erhaltenen Produkte für die vorgeschlagenen Einsatzgebiete können in bevorzugter Weise noch hochgereinigt werden. Dazu werden die semifluorierten Alkane analog [7] zunächst mit saurer Permanganatlösung behandelt, danach werden sie mit einem Gemisch von starker wäßriger Kalilauge (4-8 n), CaO bzw. BaO, und einem nukleophilen Agenz (z. B. sekundäres Amin) bei 150-180 °C ca. 72 Stunden lang am Rückfluß erhitzt oder autoklaviert. Das Umsetzungsprodukt wird abschließend mittels Scheidetrichter von der wäßrigen alkalischen Phase, die gegebenenfalls noch Alkohol enthält, und der Aminphase abgetrennt, mehrmals nacheinander mit verdünnter Mineralsäure, NaHCO₃-Lösung, destilliertem Wasser, wasserfreiem Na₂SO₄ und wasserfreiem CaCl₂ behandelt und über eine leistungsfähige Kolonne fraktioniert destilliert. Die so behandelten semifluorierten Alkane sind gemäß IR-, 1H-NMR, ¹⁹F-NMR-, GC-/MS-Spektroskopie frei von Gruppierungen, die unter intramolekularer HF-Eliminierung zur Bildung toxischer olefinischer Nebenprodukte führen könnten.
Als quantitatives Verfahren zur Bestimmung von Gruppierungen, die zur intramolekularen HF-Abspaltung oder zum Austausch eines am Kohlenstoff gebundenen Fluoratoms mittels eines nukleophilen Agenz führen können, eignet sich nach [7] die Bestimmung von ionisierbarem Fluorid bei der Reaktion des Probenmaterials mit Hexamethylendiamin in Nonan bzw. Dekan durch mehrstündiges Erhitzen bei 120 - 150 °C, wobei eventuell freigesetztes Fluorid mittels einer ionensensitiven Elektrode erfaßt wird. Nach dem Reinigungsverfahren waren demzufolge keine Fluoridionen mehr nachweisbar (Erfassungsgrenze für die Fluoridkonzentration ≤ 10⁻⁵ mol l⁻¹). Die hochgereinigten semifluorierten Alkane zeigen keine Proliferationshemmung bezüglich DNS- und Proteinsynthese an HeLa- oder Molt4- oder HEP₂-Zellkulturen. Damit sind die erfindungsgemäß genannten semifluorierten Alkane für medizinische, pharmazeutische und biologische Zwecke direkt einsetzbar.

Es ist bekannt, daß sich flüssige Perfluorcarbone aufgrund ihrer hohen Dichte und geringen Oberflächen-und Grenzflächenspannung als Behandlungsflüssigkeit zur Wiederanlegung (Entfaltung) abgelöster Netzhaut an die Aderhaut des Auges eignen [8, 9]. Allerdings eignen sich Perfluorcarbone aufgrund ihrer hohen Dichte und des damit gegebenen hohen Druckes auf die Korroidschicht nicht zur Dauertamponade.
Es ist weiterhin bekannt, daß durch die Verwendung modifizierter Perfluorcarbone der nachteilige Effekt der zu hohen Dichte gegenüber reinen Perfluorcarbonen umgangen werden kann [10], allerdings werden hierbei nur Diblock- und Triblock-Verbindungen mit R_{F} = CF₃ und C₂F₅ und R_{H} = CH₃(CH₂)ₙ mitn = 2-10 verwendet.

Es ist bekannt, zur postoperativen Tamponade eine Kombination von Perfluorphenanthren, Vitreon®, und Silikonöl zu verwenden [12]. Weil aber aufgrund der Unlöslichkeit des Perfluorcarbons im Silikonöl eine solche Kombination aus zwei nicht mischbaren Flüssigkeiten unterschiedlicher Dichten besteht, ergeben sich in dem realen System eines bewegten Auges zwangsläufig Schwierigkeiten bezüglich Durchsichtigkeit und "Emulsifizierung" an der Grenzfläche der beiden Tamponade-Flüssigkeiten.
Demgegenüber besitzen die semifluorierten Alkane des Typs R_{F}R_{H}, eine gute Löslichkeit in Silikonölen. Die semifluorierten Alkane sind umso besser in Silikonölen löslich je höher der R_{H}-Anteil ist. So sind z.B. in den für die Silikonöl-Tamponade meistens verwendeten Silikonölen mit 5000 mPa^{.}s oder 1000 mPa^{.}s die flüssigen semi-fluorierten Diblock-Verbindungen C₆F₁₃C₈H₁₇ oder C₆F₁₃C₆H₁₃ oder C₄F₉C₆H₁₃ oder C₄F₉C₅H₁₁ oder C₄F₉C₄H₉ löslich. Beispielsweise lösen sich diese R_{F}R_{H}s im Silikonöl 1000 mPa^{.}s in Verhältnissen 2:1 bis 1:2. Mit steigender Viskosität des Silikonöls nehmen die Löslichkeiten ab.
Damit ergibt sich vorzüglich eine völlig neue Anwendung für derartige homogene Lösungen von R_{F}R_{H}s in Silikonölen, aufgrund der damit gegebenen einstellbaren niederen Dichten (1,0-1,3) und der wählbaren Grenzflächen- bzw. Oberflächenspannungen (s. Tab. 3), zur Langzeit-Tamponade.
Weil die semifluorierten Alkane Lösevermittler für Perfluorcarbone sind, ist es auch möglich, Lösungen von Perfluorcarbonen in semifluorierten Alkanen, insbesondere des Types RFRH, mit den entsprechenden Silikonölen in homogene, optisch klare Systeme zu überführen und diese dann zur Tamponade zu verwenden.
Weiterhin ist mit diesen semifluorierten Alkanen, insbesondere den R_{F}R_{H}s mit relativ großem R_{H}-Anteil, ein vorzügliches Verdünnungs- und Auswaschmittel für Silikonöle nach einer Silikonöl-Netzhauttamponade gegeben. Um Silikonöle weitestgehend aus dem Auge zu entfernen, ist man bisher allein auf deren Extraktion mittels Kanüle und Spritze angewiesen.

Die semifluorierten Alkane besitzen amphiphilen Charakter. Insbesondere zeichnen sich Verbindungen des Types F(CF₂)ₙ(CH₂)ₘH durch niedrige Grenzflächenspannungen (50-58 mN/m bei 20 °C) und außerordentlich niedrige Oberflächenspannungen (15-22 mN/m bei 20 °C) aus, dadurch bedingt, daß an dem einen Molekülende die oliophoben bzw. lipophoben R_{F}-Gruppen und am anderen Ende die oliphilen bzw. lipophilen R_{H}-Gruppen gebunden sind. Dieses amphiphile, grenzflächenaktive Verhalten bewirkt, daß im Falle der Lösungen in Silikonölen an den Grenzflächen dieser Systeme sich die R_{F}R_{H}s dergestalt anordnen, daß ihr R_{H}-Teil in das Kohlenwasserstoff-haltige Lösungsmittel hineinragt, während der olephobe R_{F}-Teil nach außen geordnet ist. Eine solche Anordnung ist aber auch im Falle der Driblock-Verbindungen F(CF₂)ₙ (CH₂)ₘ(CF₂)ₙF gegeben, da in längerkettigen Monokülen durch die sterische Anordnung des Moleküls die R_{F}-Gruppen beider Enden sich einseitig gegen das langkettige Alkan-Bindeglied anordnen. Damit ergibt sich ein Sperrsicht-Effekt gegenüber dem Austreten von flüchtigen Lösungsmittel-Molekeln durch diese Grenzschichten. Wie eingangs erwähnt, nimmt die Löslichkeit in Kohlenwasserstoffen und deren Derivaten mit hohem Alkylgruppen-Anteil, und damit auch in Silikonölen, im Falle der R_{F}R_{H}s mit steigendem R_{H}-Anteil und fallendem R_{F}-Anteil zu.
Beispielsweise sind die Verbindungen C₄F₉C₄H₉ (Kp.: 129 °C), C₄F₉C₅H₁₁ (Kp.: 134 °C), C₄F₉C₆H₁₃ (Kp.: 163 °C), C₆F₁₃C₆H₁₃ (Kp.: 187 °C), C₆F₁₃C₈H₁₇ (Kp.: 223 °C) für den angegebenen Verwendungszweck und bei der angegebenen Konzentration (0,1-10 gew.%) gut löslich.

### Literatur

[1] H. Meinert, A. Knoblich, Biomat. Art. Cells & Immob. Biotech., 21 (1993) 583
[2] H. Meinert, Fluorine in Medicine in the 21st Century, Manchester (1994), paper 23
[3] R.J. Twieg et al., Macromolecules 18 (1985) 1361
[4] J. Höpken et al., Macromol. Chem. 189 (1988) 911
[5] K. von Werner, DE 39 25 525 A1 (1989)
[6] Organikum, Autorenkollektiv, Dtsch. Verlag der Wissenschaften, Berlin (1977) 363
[7] H. Meinert DE 42 05 341 A1 (1992) / WO 93/16974 A1 (1993)
[8] L.C. Clark US Pat. 4,490,351 (1984) / EP 0 112 658 A2 (1984)
[9] H. Meinert US Pat. A-5.397.805 (1995) / EP 0 493 677 A3 (1991) / DE 41 00 059 A (1994)
[10] H. Meinert DE 42 11 958 A1 (1992) / EP 0 563 446 A1 (1992)
[11] S. Chang et al., Am. J. Ophthalmol. 103 (1987) 29
   S. Chang et al., Am. J. Ophthalmol. 103 (1987) 38
   S. Chang et al., Ophthalmology 96 (1989) 785
[12] G.A. Peyman et al., Internal Tamponade in Vitreoretinal Surgery, Ravenna (1994), paper 33

## Patentansprüche

1. Verwendung eines semifluorierten Alkans der allgemeinen Formel
R_{F}R_{H}
wobei
R_{F} eine lineare oder verzweigte Perfluoralkyl-Gruppe und
R_{H} eine lineare oder verzweigte, gesättigte
(Kohlenwasserstoff)-Alkyl-Gruppe ist,
das unverzweigte semifluorierte Alkan die Formel
F(CH₂)ₙ(CH₂)ₘH
besitzt,
das verzweigte semifluorierte Alkan innerhalb der Perfluoralkyl-Gruppen -FCX- -Einheiten mit
X = C₂F₅, C₃F₇ oder C₄F₉
sowie innerhalb der Alkyl-Gruppen -HCY- -Einheiten mit
Y = C₂H₅, C₃H₇ oder C₄H₉
enthält,
innerhalb einer Perfluoralkyl-Kette eine -CX₂- -Gruppe sowie innerhalb einer Alkyl-Kette eine -CY₂- - Gruppe enthalten ist, und wobei
für alle genannten linearen oder verzweigten semifluorierten Alkane die Gesamtzahlen der Kohlenstoffatome im Perfluoralkyl-Teil von n = 3 - 20 und im Kohlenwasserstoff-Alkyl-Teil von m = 3 - 20 betragen,
zur Herstellung eines Verdünnungs- und/oder Auswaschmittels für Silikonöl nach einer Silikonöl-Netzhauttamponade oder
zur Herstellung einer Lösung des semifluorierten Alkans in Silikonöl als Hilfsmittel für die Augenheilkunde.

2. Verwendung nach Anspruch 1, wobei endständig im Molekül anstelle der Perfluoralkyl-Gruppe F₃C- eine FCX₂- oder F₂CX- -Gruppe gebunden ist und ebenso endständig im Molekül anstelle der Alkyl-Gruppe H₃C- eine HCY₂- oder H₂CY--Gruppe gebunden ist.

## Claims

1. Application of a semi-fluorinated alkane having the general formula
R_{F}R_{H}
wherein
R_{F} is a linear or branched perfluoroalkyl group, and R_{H} is a linear or branched saturated (hydrocarbon)-alkyl group;
the unbranched semi-fluorinated alkane has the formula
F(CH₂)ₙ(CH₂)ₘH;
the branched semi-fluorinated alkane comprises within the perfluoroalkyl groups -FCX- units with
X = C₂F₅, C₃F₇ or C₄F₉
and within the alkyl groups -HCY- units with
Y = C₂H₅, C₃H₇ or C₄H₉;
within a perfluoroalkyl chain there is included a -CX₂- group, and within an alkyl chain there is included a -CY₂- group; and wherein
for all said linear or branched semi-fluorinated alkanes the total numbers of carbon atoms in the perfluoroalkyl portion are within the range of n = 3 to 20 and in the hydrocarbon alkyl portion are within the range of m = 3 to 20;
for preparation of a diluting and/or rinsing agent for silicone oil after a silicone oil retina tamponade, or for preparation of a dilution of the semi-fluorinated alkane in silicone oil as an auxiliary agent in ophthalmology.

2. Application according to claim 1, wherein terminal in the molecule there is bound a FCX₂- or F₂CX- group instead of the perfluoroalkyl group F₃C-, and also terminal in the molecule there is bound a HCY₂- or H₂CY-group instead of the alkyl group H₃C-.

## Revendications

1. Utilisation d'un alcane semi-fluoré de la formule générale
R_{F}R_{H}
où
R_{F} est un groupe perfluoroalkyle linéaire ou ramifié,
R_{H} est un groupe (hydrocarbure)-alkyle saturé, linéaire ou ramifié,
l'alcane semi-fluoré linéaire possède la formule
F(CH₂)ₙ(CH₂)ₘH,
l'alcane semi-fluoré ramifié comprend des -unités -FCX- à l'intérieur des groupes perfluoroalkyle avec
X = C₂F₅, C₃F₇ ou C₄F₉,
ainsi que des -unités -HCY- à l'intérieur des groupes alkyle avec
Y = C₂H₅, C₃H₇ ou C₄H₉,
un -groupe -CX₂- étant contenu à l'intérieur d'une chaîne perfluoroalkyle, ainsi qu'un -groupe -CY₂- à l'intérieur d'une chaîne alkyle, et où
pour tous les alcanes semi-fluoré linéaires ou ramifiés cités, les nombres totaux d'atomes de carbones est de n = 3 - 20 dans la partie perfluoroalkyle, et de m = 3 - 20 dans la partie hydrocarbure-alkyle,
pour la préparation d'un agent de dilution et/ou de lavage pour l'huile de silicone, après une tamponnade de la rétine à l'huile de silicone ou
pour la préparation d'une solution de l'alcane semi-fluoré dans de l'huile de silicone en tant qu'auxiliaire pour l'ophtalmologie.

2. Utilisation selon la revendication 1, où un groupe FCX₂- ou F₂CX- est lié en partie terminale de la molécule au lieu du groupe perfluoroalkyle, et un groupe HCY₂- ou H₂CY- est lié pareillement en partie terminale de la molécule au lieu du groupe alkyle H₃C-.
